# EUROPEAN PATENT APPLICATION

(11) **EP 2 979 693 A1**
(43) Date of publication of application: **03.02.2016**
(21) Application number: 15460033.2
(22) Date of filing: 30.07.2015
(51) Int. Cl.: A61K 9/20, A61K 31/4365

(54) **PHARMACEUTICAL COMPOSITION COMPRISING PRASUGREL OR PHARMACEUTICALLY ACCEPTABLE SALT THEREOF AND PROCESS FOR ITS PREPARATION**

(30) Priority: 01.08.2014 EP 14460048
(71) Applicant: Zaklady Farmaceutyczne Polpharma SA, 83-200 Starogard Gdanski (PL)
(72) Inventor: Przerada, Szymon, 81-185 Gdynia (PL); Rzeska, Alicja, 83-200 Starogard Gdanski (PL)
(74) Representative: Chmurzynski, Sedzimir Lech

(57) **Abstract**

The present invention relates to a pharmaceutical composition comprising prasugrel or pharmaceutically acceptable salt thereof, preferably for oral administration, with increased stability and process for its preparation.

## Description

### FIELD OF THE INVENTION

The present invention relates to a pharmaceutical composition comprising prasugrel or pharmaceutically acceptable salt thereof, preferably for oral administration, with increased stability and process for its preparation.

### STATE OF THE ART

Prasugrel is a prodrug belonging to the group of thienopyridine derivatives and is an adenosine diphosphate (ADP) receptor antagonist. It is a potent inhibitor of ADP-mediated platelet aggregation in vivo and is used in the treatment or prophylaxis of thrombosis or thrombo-embolism and related diseases, and generally co-administered with acetylsalicylic acid.

Prasugrel has the chemical name 2-acetoxy-5-(alpha-cyclopropylcarbonyl-2-fluorobenzyl)-4,5,6,7-tetrahydrothieno[3,2-c]pyridine, a molecular formula C₂₀H₂₀FNO₃S, and is represented by structural Formula I.

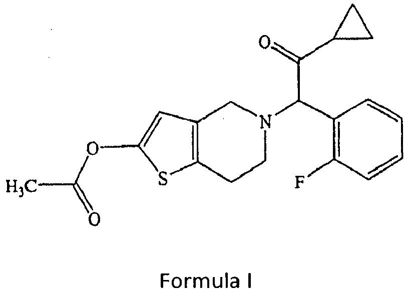

Prasugrel among other tetrahydrothienopyridine derivatives has been described for the first time in patent application EP 542411. That patent document discloses the use of these compounds for treatment and prophylaxis of thrombosis and embolism.

Prasugrel pharmaceutically acceptable salts including hydrochloride and maleate have been described in international patent application WO 02/04461. The hydrochloride salt of prasugrel is the active ingredient used in the marketed drug sold by Daiichi-Sankyo and Eli-Lilly under trade name Efient^{®}.

Prasugrel and its salts are subject to facile hydrolysis and oxidation, leading to stability problems of the active ingredient during manufacturing and storage of the formulated drug.

International patent application WO 2006/135605 describes a formulation comprising a therapeutically effective amount of prasugrel hydrochloride packed in an air and moisture impervious blister package under an inert gas to improve the stability and shelf life of prasugrel. The exemplary formulation disclosed in the application comprises prasugrel hydrochloride, mannitol, hydroxypropyl methylcellulose, croscarmellose sodium, microcrystalline cellulose and magnesium stearate.

Further, patent application US 2009/0281136 relates to a stable pharmaceutical formulation comprising prasugrel or pharmaceutically acceptable salt and discloses a pharmaceutical formulation comprising prasugrel or pharmaceutically acceptable salt thereof and at least one stabilizing agent which comprises at least one of an antioxidant and a buffer.

Furthermore, patent application EP 2275087A discloses the manufacturing process of embedding sensitive particles of prasugrel base in different protective low-melting matrices under mild conditions.

Taking into account the above solutions, there is still a need to obtain a stable pharmaceutical formulation of prasugrel or pharmaceutically acceptable salt thereof which would be manufactured in a simple and economical way without involving any special excipients such as antioxidants and special manufacturing procedures such as melt granulation.

### SUMMARY OF THE INVENTION

Surprisingly, it was found that a pharmaceutical composition comprising prasugrel or pharmaceutically acceptable salt thereof and an excipient selected from the group consisting of glycerol dibehenate, hydrogenated vegetable oil, type I, talc and mixtures thereof, preferably glycerol dibehenate, hydrogenated vegetable oil, type I, and a mixture of hydrogenated vegetable oil, type I, and talc, most preferably glycerol dibehenate or a mixture of hydrogenated vegetable oil, type I, and talc is more stable than compositions prepared with use of other excipients.

The present invention relates to a pharmaceutical composition in the form of powder composition preferably for oral administration comprising prasugrel or pharmaceutically acceptable salt thereof, which comprises an excipient selected from the group consisting of glycerol dibehenate, hydrogenated vegetable oil, type I, talc and mixtures thereof, preferably glycerol dibehenate, hydrogenated vegetable oil, type I, and a mixture of hydrogenated vegetable oil, type I, and talc, most preferably glycerol dibehenate or a mixture of hydrogenated vegetable oil, type I, and talc. Further, the invention relates to a process for preparation of a pharmaceutical composition in the form of powder composition comprising prasugrel or pharmaceutically acceptable salt thereof. The process comprises the step of blending prasugrel or pharmaceutically acceptable salt thereof with an excipient selected from the group consisting of glycerol dibehenate, hydrogenated vegetable oil, type I, talc and mixtures thereof, preferably glycerol dibehenate, hydrogenated vegetable oil, type I, and a mixture of hydrogenated vegetable oil, type I, and talc, most preferably glycerol dibehenate or a mixture of hydrogenated vegetable oil, type I, and talc.

### DETAILED DESCRIPTION OF THE INVENTION

The pharmaceutical composition in the form of powder composition according to the present invention comprises prasugrel or pharmaceutically acceptable salt thereof and an excipient selected from the group consisting of glycerol dibehenate, hydrogenated vegetable oil, type I, talc and mixtures thereof, preferably glycerol dibehenate, hydrogenated vegetable oil, type I, and a mixture of hydrogenated vegetable oil, type I, and talc, most preferably glycerol dibehenate or a mixture of hydrogenated vegetable oil, type I, and talc. Preferably the excipient is present in the composition according to the invention in an amount of 0.1% to 20%, more preferably 0.2% to 15%, most preferably 0.5% to 14%, based on the total weight of the composition.

Prasugrel is preferably used in composition of the present invention in a form of base, hydrochloride salt or maleate salt, more preferably hydrochloride salt or maleate salt, most preferably hydrochloride salt. In case of the hydrochloride salt preferably the crystalline form B1 or B2 is used, most preferably form B2 as described in EP 1298132 and in case of the maleate salt preferably the crystalline form as described in EP 1298132 is used. Preferably the prasugrel form used in composition of the present invention is micronized with PSD d(0.9) below 15µm, preferably below 12µm, most preferably below 10 µm measured with use of laser diffraction method with the following conditions:
Particle size analyzer: Mastersizer 2000, manufactured by Malvern Instruments
Dispersion unit: Hydro 2000S (wet dispersion)

| | |
|---|---|
| Analysis model: | general purpose |
| Sensitivity: | normal |
| Dispersant: | sunflower oil |
| Dispersant refractive index: | 1.47 |
| Sample refractive index: | 1.7 |
| Sample absorption index: | 0.1 |
| Single measurement time: | 5 s |
| Background measurement time: | 10 s |
| Stirrer speed: | 2000 rpm |
| Obscuration: | within 10 - 25% |
| External ultrasounds: | 1 min |

In one embodiment composition of the present invention comprises prasugrel hydrochloride and the excipient selected from the group consisting of glycerol dibehenate, hydrogenated vegetable oil, type I, talc and mixtures thereof, preferably glycerol dibehenate, hydrogenated vegetable oil, type I, and a mixture of hydrogenated vegetable oil, type I, and talc, most preferably glycerol dibehenate. The excipient is preferably used in an amount of 0.2% to 15%, more preferably 0.5% to 5%, most preferably 0.5% to 2%, based on the total weight of the composition.

In another embodiment composition of the present invention comprises prasugrel maleate and the excipient selected from the group consisting of glycerol dibehenate, hydrogenated vegetable oil, type I, talc and mixtures thereof, preferably glycerol dibehenate, hydrogenated vegetable oil, type I, and a mixture of hydrogenated vegetable oil, type I, and talc, most preferably hydrogenated vegetable oil, type I, or a mixture of hydrogenated vegetable oil, type I, and talc. The excipient is preferably used in an amount of 0.5% to 20%, more preferably 2% to 15%, most preferably 4% to 14%, based on the total weight of the composition.

In one more embodiment composition of the present invention comprises prasugrel maleat and the excipient selected from the group consisting of glycerol dibehenate, hydrogenated vegetable oil, type I, talc and mixtures thereof, preferably glycerol dibehenate, hydrogenated vegetable oil, type I, and a mixture of hydrogenated vegetable oil, type I, and talc, most preferably glycerol dibehenate. The excipient is preferably used in an amount of 0.2% to 15%, more preferably 0.5% to 5%, most preferably 0.5% to 2%, based on the total weight of the composition.

The pharmaceutical composition according to the present invention preferably comprises at least one further excipient selected from the group consisting of binders, fillers, diluents, disintegrants, glidants and lubricants, preferably binders, fillers, diluents and disintegrants.

Preferably the further suitable binders, fillers, diluents, disintegrants, glidants and lubricants are selected from excipients discussed in Handbook of Pharmaceutical Excipients, fifth edition (2006).

Preferably the powder composition according to present invention is filled into sachet or is further converted into granule, capsule or tablet, preferably capsule or tablet, most preferably tablet. Standard methods may be applied to obtain granule, capsule or tablet. For instance, granules can be obtained by roller compaction or wet granulation, preferably roller compaction, capsules by encapsulation of the powder composition in hard gelatin capsule and tablets by tableting of granules or direct compression. The most preferably the powder composition according to the present invention is further converted into tablet, preferably by direct compression. Preferably the tablet is coated with cosmetic or functional coating and is preferably packed into blister under inert gas atmosphere, more preferably nitrogen or other suitable gas.

In one embodiment of the invention the powder composition is converted into a tablet, preferably using direct compression, and the excipient selected from the group consisting of glycerol dibehenate, hydrogenated vegetable oil, type I, talc and mixtures thereof, preferably glycerol dibehenate, hydrogenated vegetable oil, type I, and a mixture of hydrogenated vegetable oil, type I, and talc, most preferably glycerol dibehenate or a mixture of hydrogenated vegetable oil, type I, and talc is used as glidant and/or lubricant, preferably as sole glidant and/or lubricant.

Further, the invention also relates to a process for preparation of a pharmaceutical composition in the form of powder composition comprising prasugrel or pharmaceutically acceptable salt thereof. The process comprises the step of blending prasugrel or pharmaceutically acceptable salt thereof with an excipient selected from the group consisting of glycerol dibehenate, hydrogenated vegetable oil, type I, talc and mixtures thereof, preferably glycerol dibehenate, hydrogenated vegetable oil, type I and a mixture of hydrogenated vegetable oil, type I, and talc, most preferably glycerol dibehenate or a mixture of hydrogenated vegetable oil, type I, and talc. The excipient is preferably used in the process according to the present invention in an amount of 0.1% to 20%, preferably 0.2% to 15%, most preferably 0.5% to 14%, based on the total weight of the composition.

Prasugrel used in the process according to the present invention is preferably used in the form of base, hydrochloride salt or maleate salt, preferably hydrochloride salt or maleate salt, most preferably hydrochloride salt. In case of the hydrochloride salt the crystalline form B1 or B2 is preferably used, most preferably B2 as described in EP 1298132 and in case of the maleate salt the crystalline form as described in EP 1298132 is preferably used. Preferably the prasugrel form used in process of the present invention is micronized with PSD d(0.9) below 12µm measured with use of laser diffraction method.

In one embodiment process of the present invention comprises use of prasugrel hydrochloride and of the excipient selected from the group consisting of glycerol dibehenate, hydrogenated vegetable oil, type I, talc and mixtures thereof, preferably glycerol dibehenate, hydrogenated vegetable oil, type I and a mixture of hydrogenated vegetable oil, type I, and talc, most preferably glycerol dibehenate. The excipient is preferably used in an amount of 0.2% to 15%, more preferably 0.5% to 5%, most preferably 0.5% to 2%, based on the total weight of the composition.

In another embodiment process of the present invention comprises use of prasugrel maleate and of the excipient selected from the group consisting of glycerol dibehenate, hydrogenated vegetable oil, type I, talc and mixtures thereof, preferably glycerol dibehenate, hydrogenated vegetable oil, type I, and a mixture of hydrogenated vegetable oil, type I, and talc, most preferably hydrogenated vegetable oil, type I, or a mixture of hydrogenated vegetable oil, type I, and talc. The excipient is preferably used in an amount of 0.5% to 20%, more preferably 2% to 15%, most preferably 4% to 14%, based on the total weight of the composition.

In one more embodiment process of the present invention comprises use of prasugrel maleate and of the excipient selected from the group consisting of glycerol dibehenate, hydrogenated vegetable oil, type I, talc and mixtures thereof, preferably glycerol dibehenate, hydrogenated vegetable oil, type I and a mixture of hydrogenated vegetable oil, type I, and talc, most preferably glycerol dibehenate. The excipient is preferably used in an amount of 0.2% to 15%, more preferably 0.5% to 5%, most preferably 0.5% to 2%, based on the total weight of the composition.

The process according to the present invention preferably comprises the use of at least one further excipient selected from the group consisting of binders, fillers, diluents, disintegrants, glidants and lubricants, preferably binders, fillers, diluents and disintegrants.

Preferably the further suitable binders, fillers, diluents, disintegrants, glidants and lubricants are selected from excipients discussed in Handbook of Pharmaceutical Excipients, fifth edition (2006).

Preferably the process according to the present invention comprises the step of filling the powder composition into sachet or further converting the powder composition into granule, capsule or tablet, preferably capsule or tablet, most preferably tablet. Standard methods may be applied to obtain granule, capsule or tablet. For instance, granules can be obtained by roller compaction or wet granulation, preferably roller compaction, capsules by encapsulation of the powder composition in hard gelatin capsule and tablets by tableting of granules or by direct compression. The most preferably the powder composition according to the present invention is further converted into tablet, preferably by means of direct compression. Preferably the tablet is coated with cosmetic or functional coating and is preferably packed into blister under inert gas atmosphere, more preferably nitrogen or other suitable gas.

In one embodiment process of the present invention comprises the steps of:
a) mixing prasugrel or pharmaceutically acceptable salt thereof with at least one excipient selected from the group consisting of binders, fillers, diluents and disintegrants;
b) adding to the mixture obtained in step (a) glidant and/or lubricant which comprises at least one excipient selected from the group consisting of glycerol dibehenate, hydrogenated vegetable oil, type I, talc and mixtures thereof, preferably glycerol dibehenate, hydrogenated vegetable oil, type I, and a mixture of hydrogenated vegetable oil, type I, and talc; most preferably glycerol dibehenate or a mixture of hydrogenated vegetable oil, type I, and talc, preferably adding to the mixture obtained in step (a) glidant and/or lubricant which consists of at least one excipient selected from the group consisting of glycerol dibehenate, hydrogenated vegetable oil, type I, talc and mixtures thereof, preferably glycerol dibehenate, hydrogenated vegetable oil, type I, and a mixture of hydrogenated vegetable oil, type I, and talc; most preferably glycerol dibehenate or a mixture of hydrogenated vegetable oil, type I, and talc;
c) blending the mixture obtained in step (b);
d) compressing the mixture of step (c) into a tablet.

Regarding used terms in the patent specification the terms mixing and blending mean the same and are used interchangeably as well as the terms mixture and blend. Term powder composition used in the patent specification is to be understood as relating to a mixture obtained by simple mixing of solid components.

Certain specific aspects and embodiments of the invention will be explained in more detail with reference to the following examples, being provided only for purpose of illustration, and it is to be understood that the present invention should not be deemed to be limited there to.

### Example 1

| | [%/tablet] |
|---|---|
| Prasugrel hydrochloride (form B2) | 5.50 |
| Microcrystalline cellulose (PH 102) | 22.00 |
| Croscarmellose sodium (Ac-di-sol) | 2.00 |
| Mannitol (100 SD) | 66.00 |
| Hydroxypropyl cellulose (Klucel LF) | 2.50 |
| Glycerol dibehenate (Compritol 888 ATO) | 2.00 |

The weighed amount of prasugrel hydrochloride, microcrystalline cellulose, croscarmellose sodium, mannitol and hydroxypropyl cellulose are sieved through 0.8mm sieve. That obtained mixture is blended for 30 min. with use of bin tumbler (Kates ML-B1109) (12 rotation/min). After that the weighed amount of glycerol dibehenate is added and the resulting mixture is blended for additional 10 min with use of the same conditions. The resulting mixture is compressed into a tablet with use of single punch tablet press (Korsch XP 1) and then coated with a cosmetic coating, with use of drum coater (Glatt GC 300EX) The resulting tablets have the weight of 100mg per tablet before coating.

### Example 2

| | [%/tablet] |
|---|---|
| Prasugrel hydrochloride (form B2) | 5.50 |
| Microcrystalline cellulose (PH 102) | 22.00 |
| Croscarmellose sodium (Ac-di-sol) | 2.00 |
| Mannitol (100 SD) | 64.00 |
| Hydroxypropyl cellulose (Klucel LF) | 2.50 |
| Hydrogenated vegetable oil, type I, (Lubritab) | 1.00 |
| Talc | 3.00 |

Procedure identical to that of Example 1 with exception that instead of glycerol dibehenate hydrogenated vegetable oil, type I, and talc are used and sieved through 0.8mm sieve. The resulting tablets have the weight of 100mg per tablet before coating.

### Example 3

| | [%/tablet] |
|---|---|
| Prasugrel hydrochloride (form B2) | 5.50 |
| Microcrystalline cellulose (PH 102) | 22.00 |
| Croscarmellose sodium (Ac-di-sol) | 2.00 |
| Mannitol (100 SD) | 66.00 |
| Hydroxypropyl methylcellulose (Methocel E5) | 2.50 |
| Glycerol dibehenate (Compritol 888 ATO) | 2.00 |

Procedure identical to that of Example 1 with exception that instead of hydroxypropyl cellulose hydroxypropyl methylcellulose is used. The resulting tablets have the weight of 100mg per tablet before coating.

### Example 4

| | [%/tablet] |
|---|---|
| Prasugrel maleate | 6.53 |
| Microcrystalline cellulose (PH 102) | 20.99 |
| Croscarmellose sodium (Ac-di-sol) | 2.00 |
| Mannitol (100 SD) | 65.98 |
| Hydroxypropyl cellulose (Klucel LF) | 2.50 |
| Hydrogenated vegetable oil, type I, (Lubritab) | 1.00 |
| Talc | 1.00 |

Procedure identical to that of Example 1 with exception that instead of prasugrel hydrochloride prasugrel maleate is used and instead of glycerol dibehenate hydrogenated vegetable oil, type I, and talc are used and sieved through 0.8mm sieve. The resulting tablets have the weight of 100mg per tablet before coating.

### Example 5

| | [%/tablet] |
|---|---|
| Prasugrel maleate | 6.53 |
| Microcrystalline cellulose (PH 102) | 21.99 |
| Croscarmellose sodium (Ac-di-sol) | 2.00 |
| Mannitol (100 SD) | 65.98 |
| Hydroxypropylmethyl cellulose (Methocel E5) | 2.50 |
| Glycerol dibehenate (Compritol 888 ATO) | 1.00 |

Procedure identical to that of Example 1 with exception that instead of prasugrel hydrochloride prasugrel maleate is used and instead of hydroxypropyl cellulose hydroxypropylmethyl cellulose is used. The resulting tablets have the weight of 100mg per tablet before coating.

### Example 6 - comparative example

| | [%/tablet] |
|---|---|
| Prasugrel hydrochloride (form B2) | 5.50 |
| Microcrystalline cellulose (PH 102) | 22.00 |
| Croscarmellose sodium (Ac-di-sol) | 2.00 |
| Mannitol (100 SD) | 67.00 |
| Hydroxypropyl cellulose (Klucel LF) | 2.50 |
| Magnesium stearate | 1.00 |

Procedure identical to that of Example 1 with exception that instead of glycerol dibehenate magnesium stearate is used. The resulting tablets have the weight of 100mg per tablet before coating.

### Example 7 - comparative example

| | [%/tablet] |
|---|---|
| Prasugrel maleate | 6.53 |
| Microcrystalline cellulose (PH 102) | 21.99 |
| Croscarmellose sodium (Ac-di-sol) | 2.00 |
| Mannitol (100 SD) | 65.98 |
| Hydroxypropyl cellulose (Klucel LF) | 2.50 |
| Magnesium stearate | 1.00 |

Procedure identical to that of Example 1 with exception that instead of prasugrel hydrochloride prasugrel maleate is used and instead of glycerol dibehenate magnesium stearate is used. The resulting tablets have the weight of 100mg per tablet before coating.

The resulting tablets as obtained in Examples 1-7 were packed into AI/AI blisters under nitrogen atmosphere. All the tablets were put into stability tests (40°C/75%RH). The results of the tests are shown in the table below:

**Table 1 Results of the stability tests (40°C/75%RH)**

| | Example 1 | Example 2 | Example 3 | Example 4 | Example 5 | Example 6 comparative | Example 7 comparative |
|---|---|---|---|---|---|---|---|
| Impurity level time 0 [%] | 0.40 | 0.47 | 0.50 | 0.17 | 0.15 | 0.45 | 0.48 |
| Impurity level after 1 month [%] | 0.98 | 1.11 | 0.75 | 0.58 | 0.52 | 1.25 | 1.32 |
| Impurity level after 3 months [%] | 1.01 | 1.62 | 1.27 | 1.46 | 1.64 | 1.86 | NT* |

| | | | | | | | |
|---|---|---|---|---|---|---|---|
| * not tested | | | | | | | |

The level of impurity was tested with use of HPLC method with the following conditions:

| | |
|---|---|
| Column: | C18 (Inertsil ODS-2 150 A 150x4,6 mm, 5 µm) |
| Mobile phase: | phase A: buffer pH 2,9 |
| | phase B: acetonitrile: water 90:10 (V/V) |
| Flow rate: | 1,0 ml/min; |
| Detection: | 235 nm; |
| Injected amount: | 20 µl |
| Run time: | 60min |
| Diluent: | acetonitrile: water 90:10 (V/V) |

## Claims

1. A pharmaceutical composition comprising prasugrel or pharmaceutically acceptable salt thereof, wherein the composition is in the form of powder composition and comprises an excipient selected from the group consisting of glycerol dibehenate, hydrogenated vegetable oil, type I, talc and mixtures thereof, preferably glycerol dibehenate, hydrogenated vegetable oil, type I, and a mixture of hydrogenated vegetable oil, type I, and talc, most preferably glycerol dibehenate or a mixture of hydrogenated vegetable oil, type I, and talc.

2. The pharmaceutical composition according to claim 1, wherein the excipient is present in the composition in an amount of 0.1 to 20%, preferably 0.2 to 15%, most preferably 0.5 to 14%, based on the total weight of the composition.

3. The pharmaceutical composition according to any of the preceding claims, wherein prasugrel is in the form of base, hydrochloride salt or maleate salt, preferably hydrochloride salt or maleate salt, most preferably hydrochloride salt.

4. The pharmaceutical composition according to any of the preceding claims, wherein the composition comprises at least one further excipient selected from the group consisting of binders, fillers, diluents, disintegrants, glidants and lubricants, preferably binders, fillers, diluents and disintegrants.

5. The pharmaceutical composition according to any of the preceding claims, wherein the powder composition is further converted into granule, capsule or tablet, preferably capsule or tablet, most preferably tablet.

6. The pharmaceutical composition according to claim 5, wherein the composition is in the form of a tablet, preferably prepared by direct compression.

7. The pharmaceutical composition according to claim 6, wherein the excipient selected from the group consisting of glycerol dibehenate, hydrogenated vegetable oil, type I, talc and mixtures thereof, preferably glycerol dibehenate, hydrogenated vegetable oil, type I and a mixture of hydrogenated vegetable oil, type I, and talc, most preferably glycerol dibehenate or a mixture of hydrogenated vegetable oil, type I, and talc is used as glidant and/or lubricant.

8. A process for the preparation of a pharmaceutical composition in the form of powder composition comprising prasugrel or pharmaceutically acceptable salt thereof, wherein the process comprises the step of blending prasugrel or pharmaceutically acceptable salt thereof with an excipient selected from the group consisting of glycerol dibehenate, hydrogenated vegetable oil, type I, talc and mixtures thereof, preferably glycerol dibehenate, hydrogenated vegetable oil, type I, and a mixture of hydrogenated vegetable oil, type I, and talc, most preferably glycerol dibehenate or a mixture of hydrogenated vegetable oil, type I, and talc.

9. The process according to claim 8, wherein the excipient is used in an amount of 0.1 to 20%, preferably 0.2 to 15%, most preferably 0.5 to 14%, based on the total weight of the composition.

10. The process according to claim 8 or 9, wherein prasugrel is used in the form of base, hydrochloride salt or maleate salt, preferably hydrochloride salt or maleate salt, most preferably hydrochloride salt.

11. The process according to claim 8, 9 or 10, wherein the process comprises admixing with the blend at least one further excipient selected from the group consisting of binders, fillers, diluents, disintegrants, glidants and lubricants, preferably binders, fillers, diluents and disintegrants.

12. The process according to claim 8, 9, 10 or 11, wherein the process further comprises the step of converting the powder composition into granule, capsule or tablet, preferably capsule or tablet, most preferably tablet.

13. The process according to claim 12, wherein the process results in obtaining a tablet, preferably by means of direct compression.

14. The process according to claim 13, wherein the excipient selected from the group consisting of glycerol dibehenate, hydrogenated vegetable oil, type I, talc and mixtures thereof, preferably glycerol dibehenate, hydrogenated vegetable oil, type I, and a mixture of hydrogenated vegetable oil, type I, and talc, most preferably glycerol dibehenate or a mixture of hydrogenated vegetable oil, type I, and talc is used in the process as glidant and/or lubricant.

15. A process for preparation of the pharmaceutical composition according to claim 6 or 7, wherein the process comprises the steps of:
a) mixing prasugrel or pharmaceutically acceptable salt thereof with at least one excipient selected from the group consisting of binders, fillers, diluents and disintegrants;
b) adding to the mixture obtained in step (a) glidant and/or lubricant which comprises at least one excipient selected from the group consisting of glycerol dibehenate, hydrogenated vegetable oil, type I, talc and mixtures thereof, preferably glycerol dibehenate, hydrogenated vegetable oil, type I, and a mixture of hydrogenated vegetable oil, type I, and talc, most preferably glycerol dibehenate or a mixture of hydrogenated vegetable oil, type I, and talc;
c) blending the mixture obtained in step (b);
d) compressing the mixture of step (c) into a tablet.
